# EUROPEAN PATENT APPLICATION

(11) **EP 4 740 994 A1**
(43) Date of publication of application: **13.05.2026**
(21) Application number: 24212253.9
(22) Date of filing: 12.11.2024
(51) Int. Cl.: A61M 39/10, A61M 39/12, A61M 39/20, A61J 1/14

(54) **A SMALL-BORE FEMALE CONNECTOR FOR ENGAGING A FLEXIBLE TUBE**

(71) Applicant: B. Braun Melsungen AG, 34212 Melsungen (DE)
(72) Inventor: VALLOTTON, Raphael, 34212 Melsungen (DE)
(74) Representative: Maiwald GmbH

(57) **Abstract**

A small-bore female connector suitable for being inserted into a flexible tube, wherein the small-bore female connector comprises a hollow body part, a first removable part, and a second removable part, wherein the hollow body part comprises a device-side part comprising a small-bore female connector part having a small-bore female opening, and a tube-side part comprising a tube engaging part having a fluid receiving opening, wherein the first removable part forms a tube-side connection to the tube-side part of the hollow body part, thereby breakably closing the fluid receiving opening, and wherein the second removable part forms a device-side connection to the device-side part of the hollow body part, thereby breakably closing the small-bore female opening.

## Description

### Technical Field of the Invention

The present invention relates to a small-bore female connector, in particular a small-bore female connector which can be inserted into a flexible tube, and which has a simple, easy to manufacture design. Furthermore, the present invention relates to the use of such a small-bore female connector in a pharmaceutical product and a process for preparing such a small-bore female connector.

### Background of the Invention

Pharmaceutical ingredients have an intended application purpose, for which they need to be inserted in specific downstream devices. Hence, pharmaceutical ingredients and solutions thereof are commonly stored prior to use and are transferred to respective downstream devices or storage containers shortly before usage.

Typically, such pharmaceutical solutions are stored in glass or plastic vials, glass or plastic ampoules, or plastic pouches. Historically, vials, glass bottles, and plastic pouches were accessed through elastomeric stoppers using needles, spikes, or other types of sharp transfer devices for puncturing said elastomeric stoppers. Accessing vials, ampoules or pouches and transferring the pharmaceutical solution using needles has the drawback of potential risks using sharp devices, such as piercing of the container walls, creation of fragments of elastomeric stopper or plastic membrane, needle-stick injuries, and general difficulty of pushing the needle through the elastomeric stopper.

To overcome the problem of handling the solution with a needle, other kinds of containers such as glass ampoules and later plastic ampoules were developed over the years, which provide access to their content through an open neck after breaking off an ampoule head. Such ampoules can be accessed using a needle, similar to those used to pierce elastomeric stoppers, or blunt needles. In addition, also plastic needles of transfer devices, as well as semi-flexible straws could be used to transfer the pharmaceutical solution. Moreover, for withdrawing the content of glass ampoules, needles or straws are typically equipped or combined with inline filters to prevent glass particles from being directed into the withdrawing devices and later being administered to the patient.

Needle-free connectors have been developed to overcome the above-mentioned problems, cf. WO 9600556 A1. The most common needle-free connector for intravenous application is the LUER connector, as currently defined in ISO 80369-7.

This connector allows liquid-tight connection of two devices and transfer of a pharmaceutical solution in between these devices. Likewise, an example for a connector for neuraxial application is the NRFit^{®} connector, as currently defined in ISO 80369-6.

It is generally a desire to provide needle-free connectors having a sealing function. In this way, complicated designs including further means for sealing the connection could be avoided.

However, generally, female LUER or NRFit connectors have a small fluid conduit, wherein the diameter of this conduit is in the range of a few millimeters. Such small diameters make it difficult to position an elastomeric resealing member to achieve a sealing function. While LUER-activated valves are known from the prior art, the design of some features of such LUER-activated valves deviates from the dimensions stated in ISO 80369-7 in order to give enough room for the elastomeric and its deformation (i.e. the threads supporting diameter and its axial extend, as well as the *'functional'* inner taper of the LUER cone receiving the male mating connector). This is in particular true for the length of the outer threaded portion and the depth of the tapered female portion (cf. AAMI CN27). These modifications can have a negative impact on the seat of the male connector in the female connector and generally on the connection strength and tightness. NRFit connectors have an even smaller inner diameter of the fluid conduit, which makes it hardly possible to place an elastomeric member therein. Hence, even larger deviations from nominal dimensions as stated in ISO 80369-6 would be necessary to achieve an NRFit-activated valve.

### Summary of the Invention

Hence, the small-bore female connector activated valves known from the prior art have the disadvantage that they deviate from the standards risking problems of the connection, i.e. reduced strength and tightness. On the other hands, standard-conform small-bore female connectors known from the prior art have the disadvantage that they require additional means for sealing.

Hence, the object of the present invention is to find a small-bore connector, in particular a female small-bore connector, which allows for sealing of a conduit until a used defined opening event and which allows for completely fulfilling the standards for said small-bore connector. Furthermore, it is another object of the present invention to find a small-bore connector, in particular a female small-bore connector, which ensures that an inner cavity or conduit enclosed by said small-bore connector stays sterilized after sterilization.

It has now surprisingly been found that above-mentioned object is achieved by a small-bore female connector suitable for being inserted into a flexible tube, wherein the small-bore female connector comprises
- a hollow body part,
- a first removable part, and
- a second removable part,

wherein the hollow body part comprises
   - a device-side part comprising a small-bore female connector part having a small-bore female opening, and
   - a tube-side part comprising a tube engaging part having a fluid receiving opening,
wherein the first removable part forms a tube-side connection to the tube-side part of the hollow body part, thereby breakably closing the fluid receiving opening, and
wherein the second removable part forms a device-side connection to the device-side part of the hollow body part.

Moreover, it has now surprisingly been found that above-mentioned object is also achieved by a process for preparing a small-bore female connector according to the present invention, wherein the process comprises the steps of:
- injection molding the small-bore female connector thereby shaping the tube-side connection and the device-side connection;
- closing the twist-off opening of the second removable part.

Finally, it has surprisingly been found that above-mentioned object is achieved by the use of the small-bore female connector according to the present invention in a pharmaceutical product, preferably as a port in a pharmaceutical product, more preferably as a port in a bag comprising an active pharmaceutical ingredient.

The present invention has the advantage that the small-bore female connector is liquid-tight until activated. This is achieved by providing the first removable part at the end of the connector which can be inserted into an engaging tube. After tightly connecting a male connector of another device into the small-bore female connector according to the invention, the user can break the first removable part by bending the tube. Another advantage of the small-bore female connector is that it can be manufactured from only one singly molded component. Such a design is significantly cheaper than connectors comprising a LUER-activated valve. Another advantage of the small-bore female connector is that the inner conduit is sealed and remains sterile until use if sterilized (for example by radio sterilization) after sealing the twist-off opening, hence does not require disinfection before connecting a mating device to transfer fluid through said connector.

### Short Description of the Figures

- Figure 1: is a schematic drawing of the most preferred embodiment of the present invention.
- Figure 2: is a schematic drawing of the most preferred embodiment of the present invention including cap.
- Figure 3: is a schematic drawing of the most preferred embodiment of the present invention with open and with closed, i.e. sealed, twist-off opening.

### List of reference signs

- **1**: Hollow body part
- **2**: First removable part
- **3**: Second removable part
- **4**: Device-side part
- **5**: Small-bore female connector part
- **6**: Small-bore female opening
- **7**: Tube-side part
- **8**: Tube engaging part
- **8a**: engaging section of tube engaging part
- **8b**: tapered part of tube engaging part
- **9**: Fluid receiving opening
- **10**: Tube-side connection
- **11**: Device-side connection
- **12**: Radially extending flange
- **13**: Hole
- **14**: Lateral extensions of first removable part
- **15**: Lateral extensions of second removable part
- **16**: Twist-off opening
- **17**: Retaining string
- **18**: Cap

### Definitions

The term *'liquid-tight'* as used herein denotes a quality of an object to function as a barrier for a liquid, preferably for a water-based liquid.

The term *'sterile'* as used denotes the status of an object having a significantly reduced number of bacteria and/or viruses on its surface to reduce the risk of an infection. In particular, the term *'sterile'* denotes an object or substance, which has a bioburden load of lower than 10⁻⁶. The bioburden load can be measured i.e., according to ISO 11737-1:2018.

The term *'sterilization'* as used herein denotes a method to destroy all forms of living microorganisms from a substance. As there is always a certain probability of at least one microorganism to survive such procedure, the aim of sterilization is the reduction of initially present microorganisms or other potential pathogens. Generally, sterilization is accepted to be achieved if the bioburden load of the substance of object to be sterilized is lower than 10⁻⁶. The bioburden load can be measured i.e., according to ISO 11737-1:2018. Sterilization can be achieved using several methods. In one sterilization process the object is heated up to at least 105 °C to achieve a sterile object. Thereby, the object should not be deformed by the elevated temperature. Preferably, the heating step is performed in an autoclave. In another sterilization process, the object is brought into contact with toxic gases such as a mixture of ethylene oxide and carbon dioxide. Filtration methods are also used to sterilize liquids, i.e., by using membrane filters, Seitz filters, and/or candle filters. Finally, sterilization can be achieved by indirect energy import into or onto the object, e.g., by ultrasonic waves, ultraviolet light, as well as by high energy particles (such as electrons, gamma- or X-rays).

The term *'heat sealing'* as used herein denotes thermal welding of polymer components. In this process, at least two polymer components are heated until at least the glass transition temperature is reached and the plastics in these areas begin to become liquid or at least low viscous. By simultaneous or subsequent mechanical pressing of these areas, the polymers are mixed together at this point and, after cooling and solidification, form a fixed joint. Heat sealing a tube may involve pre-heating the inner surface of said tube to soften or melt said inner surface while preventing the outer surface to melt, hence reduce the risk of sticking onto the tools used to press the tub to achieve the tight seal.

The term *'polymer'* as used herein denotes an organic polymer, preferably a polymer selected from polyolefins, polyethylene terephthalate, polystyrene, polyvinyl chloride, or mixtures thereof, more preferably a polyolefin selected from the list consisting of polyethylene, polypropylene, or mixtures thereof.

The term *'additive'* as used herein denotes further components, which can be present in polymer compositions to modify their physical properties. Examples of additives are antioxidant(s), stabilizer(s), such as process stabilizers and UV stabilizers, acid scavenger(s), metal deactivators, crosslinking agents, such as free radical generating agent(s), e.g., organic peroxide(s), scorch retarder(s), crosslinking booster(s), processing aid(s), flame retardant additive(s), water tree retardant additive(s), inorganic filler(s), and voltage stabilizer(s). These groups of additives and the individual additive compounds therein are usually well known in the polymer field.

### Detailed Description of the Invention

As set out above, the present invention concerns a small-bore female connector, a process for preparing such a small-bore female connector according to the present invention and the use of such a small-bore female connector according to the present invention, each of which will be described in detail in the following.

### Small-bore female connector

As set out above, the most general embodiment of the present invention refers to a small-bore female connector suitable for being inserted into a flexible tube, wherein the small-bore female connector comprises
- a hollow body part (**1**),
- a first removable part (**2**), and
- a second removable part (**3**),

wherein the hollow body part (**1**) comprises
   - a device-side part (**4**) comprising a small-bore female connector part (**5**) having a small-bore female opening (**6**), and
   - a tube-side part (**7**) comprising a tube engaging part (**8**) having a fluid receiving opening (**9**),
wherein the first removable part (**2**) forms a tube-side connection (**10**) to the tube-side part (**7**) of the hollow body part (**1**), thereby breakably closing the fluid receiving opening (**9**), and
wherein the second removable part (**3**) forms a device-side connection (**11**) to the device-side part (**4**) of the hollow body part (**1**).

Preferably, the small-bore female connector is selected from the list consisting of a small-bore female connector according to ISO 80369-7, a small-bore female connector according to ISO 80369-6, and a small-bore female connector according to ISO 80369-3, for intravascular, neuraxial, and enteral application, respectively. These small-bore female connectors have the advantage that they are commonly used and therefore ensure broad and convenient compatibility with downstream devices or storage containers. More preferably, the small-bore female connector according to the present invention is a small-bore female connector according to ISO 80369-7.

Hence, preferably, the small-bore female connector according to the present invention is compatible with a male connector according to ISO 80369-7, compatible with a male connector according to ISO 80369-6, or compatible with a male connector according to ISO 80369-3. An example for a connector according to ISO 80369-7 is the so-called LUER connector. Likewise, an example for a connector according to ISO 80369-6 is the NRFit^{®} connector and an example for a connector according to ISO 80369-3 is the ENFit^{®} connector, both tradenames registered by GEDSA.

The small-bore female connector according to the present invention is preferably prepared by a preparation method selected from the list consisting of additive manufacturing, such as 3D printing, , extrusion blow molding, or injection molding. Preferably, the small-bore female connector according to the present invention is prepared by injection molding.

In the extrusion blow molding process, plastic material is extruded in a parison, preferably a tube-shaped parison. The parison is inserted into a mold, preferably in that the mold comprises, preferably consists of two or more parts, which are disassembled (i.e. opened) before inserting the parison, and which are assembled (i.e. closed) after insertion of the parison. The mold preferably has a shape in that the assembled mold closes the tube-shaped parison at one end and allows for inserting pressurized gas, preferably air, into the tube-shaped parison via the other end allowing for inflating the parison onto an inner surface of the mold. The mold preferably cools the extruded material of the parison. The advantage of extrusion blow molding is that the process step is simple and cheap. Another advantage is, even if not often used yet, that by extrusion multilayered materials can be obtained.

In injection molding, a polymer is injected in molten form into a mold. In the injection molding process for preparing the small-bore female connector according to the present invention, in the molding step a central pin is partially retracted to increase the wall thickness of the tube-side connection and the device-side connection. Subsequently, the pin is then pushed forward before the polymer melt freezes, in order to reduce said wall thickness to a value which makes it possible to remove the attached first or second removable part manually. Such a process is usually called injection-compression molding.

Preferably, the small-bore female connector is made from a polymer material. The polymer material is preferably selected from the list consisting of polyethylene, polypropylene, polycarbonate, polyvinylchloride, and mixtures thereof. More preferably the polymer material is selected from polyethylene or polypropylene. These materials have an ideal balance of material properties, processability, inertia, and low price. Additionally, the polymer material can preferably comprise one or more additives.

Preferably, the polymer material has a glass transition temperature *T_{g}* measured according to ASTM D3418, of not more than 80 °C, preferably not more than 60 °C, more preferably not more than 40 °C, and most preferably not more than 20 °C. This ensures thermal stability in particular in view of the sterilization conditions used.

As the typical application of the small-bore female connector requires sterile environments, the small-bore female connector is preferably sterile. More preferably, the small-bore female connector has been sterilized, most preferably radio sterilized.

### Hollow body part

In the small-bore female connector according to the present invention, the hollow body part (**1**) preferably further comprises a flange (**13**), preferably a radially extending flange (**12**). More preferably, the radially extending flange (**12**) is positioned at a connection between the tube-side part (**7**) and the device-side part (**4**). Thus, the flange (**12**) is preferably located at the tube-side part (**7**) opposite of the first removable part (**3**). In such a way, the flange functions as a stopping means for the tube engaging part (**8**).

Furthermore, in the small-bore female connector according to the present invention the hollow body part (**1**) comprises a hole (**13**) extending through the hollow body part (**1**) connecting the small-bore female opening (**6**) and the fluid receiving opening (**9**). This hole (**13**) functions as a fluid pathway. Furthermore, such a hole facilitates preparation by an extrusion blow molding process as well as by an injection molding process.

In a further preferred embodiment of the small-bore female connector according to the present invention the tube engaging part (**8**) comprises a engaging section (**8a**) for engaging with the tube and a tapered, preferably conical, section (**8b**), wherein the engaging section (**8a**) connects the tube engaging part (**8**) to the hollow body part (**1**), and wherein the tapered part (**8b**) connects the engaging section (**8a**) with the first removable part (**2**). Preferably, the engaging section (**8a**) is also a tapered, preferably conical, section, wherein the taper angle of the engaging section (**8a**) is lower, i.e. flatter, than the taper angle of the tapered section (**8b**). Most preferably, however, the engaging section (**8a**) is a cylindrical section. This design facilitates engaging and centering the tube.

In another preferred embodiment, the small-bore female connector according further comprises a retaining string (**17**), wherein the retaining string (**17**) is connected to the second removable part (**3**) and the hollow body part (**1**), preferably the flange (**12**) of the hollow body part (**1**). This design has the advantage of keeping the second removable part (3) attached to the connector once detached. Thus, it is avoided that it falls-off or has to be thrown away by the user.

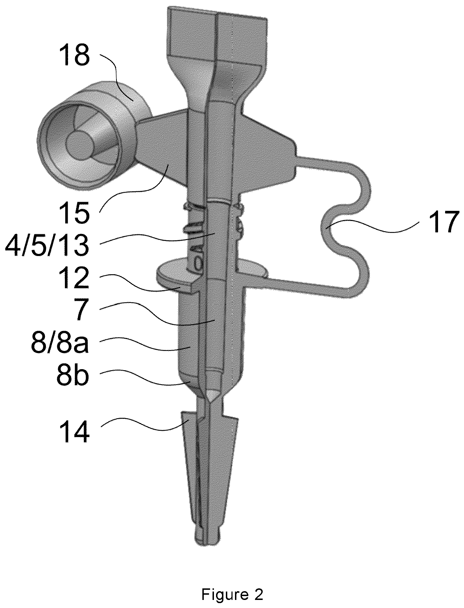

Furthermore, in another preferred embodiment, the small-bore female connector further comprises a cap (**18**) suitable for being placed on the small-bore female connector part (**5**) after twisting off the second removable part (**3**) (cf. Figure 2). The cap can be used to close the connector after using the connector. This reduces or prevents leaking. Preferably, the cap (**18**) is connected to the second removable part (**3**). Most preferably, the cap (**18**) is connected to the second removable part (**3**) and the small-bore female connector according further comprises a retaining string (**17**), wherein the retaining string (**17**) is connected to the second removable part (**3**) and the hollow body part (**1**), preferably the flange (**12**) of the hollow body part (**1**). This prevents losing the cap.

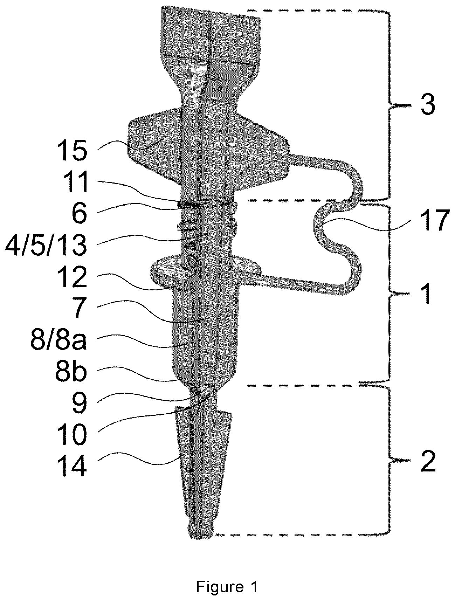

In the most preferably embodiment of the present invention, the small-bore female connector comprises all of the preferred features described beforehand. Such an embodiment is depicted in Figures 1 and 2.

### First removable part

First removable parts are provided in several types of axial connectors for medical use to allow for insertion in flexible tubes. Such connectors are sometimes provided with caps to protect the fluid path from contamination before use. The disadvantage of this solution is that it requires a second molded part and an additional assembly step. To ensure tightness of the fluid path before use and resealing after use, the prior art also offers similar ports with a LUER-activated valve. This adds the already described disadvantages. Moreover, in some applications, while tightness and integrity before use is required, resealing after use is not necessarily required, i.e. leakages after use are acceptable. In those cases, using a port having an elastomeric valve would further have the disadvantage of increased cost for no added value.

To establish a fluid connection through the small-bore female connector according to the present invention, the first removable part (**2**) needs be bent so as to rupture the tube-side connection (**10**) that connects the tube-side part (**7**) and the first removable part (**2**). The broken-off first removable part (**2**) will then be separated a few millimeters from the tube-side part (**7**). This allows fluid to pass and flow from one end of the small-bore female connector to the other. Hence, preferably, the first removable part (**2**) is a break-off part.

Hence, preferably, in the small-bore female connector according to the present invention, the tube-side connection (**10**) is liquid-tight and breakable by application of a lateral force and/or torque. Preferably, the force is a force applicable by hand.

In preferred embodiment of the present invention, the first removable part (**2**) comprises at least two lateral extensions (**14**). This has the advantage that the first removable part (**2**) is in direct connection with the tube, helps centering the tube during the engaging part and applies the lateral or torque force directly from the tube to the first removable part (**2**).

Even more preferably, the breadth of the at least two lateral extensions (**14**) decreases along the lateral dimension of the at least two lateral extensions (**14**) from the tube-side part (**7**) towards an end of the first removable part (**2**). This design has the advantage that while bending the tube to break-off the first removable part (**2**) said part is moved away from the tube-side part (**7**), manages a series of fluid channel extending from the hollow body part (**1**) and the tube, passing between the at least two lateral extensions (**14**), and the lateral extensions (**14**) act as hooks which prevent that the broken-off first removable part (**2**) moves back towards the tube-side part (**7**). Hence, the risk of blockage of the flow through the small-bore female connector is reduced. Most preferably, at least one edge of the lateral extensions (**14**) is sharp. Preferably, at least one edge of the lateral extensions (**14**) pointing towards the tube-side part (**7**) is sharp. This design has the advantage that the broken-off first removable part (**2**) is hooked in place and does allow movement of the first removable part (**2**) only in one direction, i.e. away from the tube-side part (**7**).

### Second removable part

At the device-side part (**4**) of connectors known from the prior art, in particular the small-bore female connector part, there is a risk of contaminating particulate matter and/or biological contaminants without any protection. While such connectors can easily be provided with a suitable cap, such a cap is an additional component that needs to be molded separately and later assembled on the connector, leading to a more complex and more costly manufacturing process. Furthermore, said cap can come loose and fall from the connector, before, as well as after exposing the fluid path of the connector.

To overcome this problem, the small-bore female connector of the present invention comprises a second removable part (**3**). Thereby, the device-side part (**4**) of the hollow body part (**1**) are connected by the device-side connection (**11**), which can be a thinned annular connection, preferably an annular area having a reduced thickness in comparison to the device-side part (**4**).

The second removable part (**3**) can be removed by applying a bending or twisting movement thereby exposing the fluid path of the connector. Hence, preferably, the device-side connection (**11**) of the small-bore female connector according to the present invention is liquid-tight and breakable by application of a lateral force or torque. Hence, preferably, the second removable part (**3**) is a twist-off part.

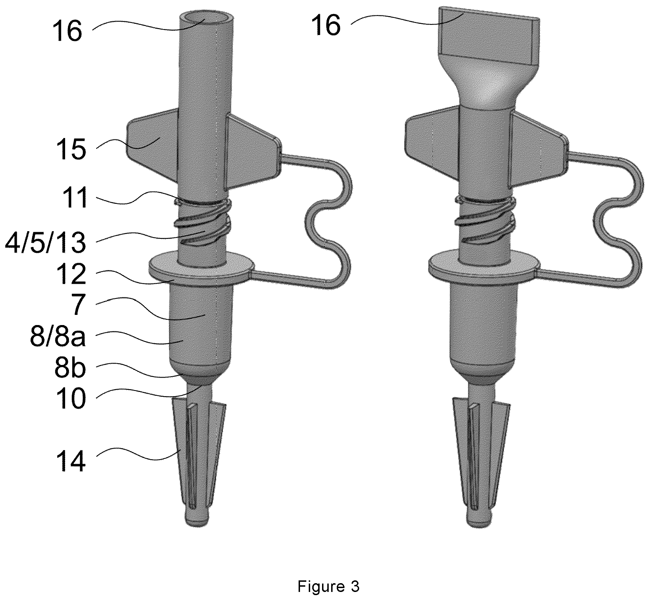

Preferably, in the small-bore female connector according to the present invention, the second removable part (**3**) forming a device-side connection (**11**) to the device-side part (**4**) of the hollow body part (**1**) results in breakably closing the small-bore female opening (**6**). This can be achieved for example by the second removable part being a solid component, i.e. not having a hole at all. However, as set out above, preferably, the small-bore female connector is prepared by an extrusion blow-molding process or an injection molding process. However, an extrusion blow molding process or an injection molding process requires that the formed shape is hollow and comprises at least one hole. Preferably, this hole is present in the second removable part (**3**). Hence, preferably, in the small-bore female connector according to the present invention the second removable part (**3**) comprises a twist-off opening (**16**). Such a twist-off opening (**16**) is for example disclosed in Figure 3. On the left side of Figure 3 the second removable part is depicted having a still open twist-off opening (**16**), wherein on the right side of Figure 3 the final small-bore female connector can be seen, in which the twist-off opening has been sealed. Hence, preferably, in the small-bore female connector according to the present invention the twist-off opening (**16**) is closed, preferably with a plug or sealed, most preferably sealed.

Furthermore, preferably, in the small-bore female connector according to the present invention the second removable part (**3**) comprises at least two lateral extensions (**15**) for gripping said second removable part (**3**) and applying force or torque to twist it off. This facilitates prehension to apply twist-off force, bending and/or torsion.

### Use

The present invention further relates to the use of the small-bore female connector according to the invention in a pharmaceutical product, preferably as a port in a pharmaceutical product, more preferably as a port in a bag comprising an active pharmaceutical ingredient. Preferably, the method of using the small-bore female connector according to the invention in a pharmaceutical product comprises the steps of:
- Removing the second removable part (**2**) thereby exposing the small-bore female connector part (**5**);
- Connecting a small-bore male connector of a suitable device to small-bore female connector part (**5**);
- Transferring a pharmaceutical compound from the pharmaceutical product to the medical device of from the device to the pharmaceutical product through the small-bore connectors.

Preferably the device of the method of using the small-bore female connector according to the present invention is a medical device or a pharmaceutical device.

### Process

Finally, the present invention is related to a process for preparing a small-bore female connector according to the present invention, wherein the process comprises the steps of:
- molding the small-bore female connector thereby shaping the tube-side connection (**10**) and the device-side connection (**11**);
- closing the twist-off opening (**15**) of the second removable part (**3**).

Preferably, the step of closing of the twist-off opening (**16**) comprises the step of inserting a plug into the twist-off opening (**16**) or the step of sealing, preferably thermally sealing, most preferably heating, flattening and welding, the twist-off opening (**16**).

The molding step can be an extrusion blow molding step, or an injection blow molding step.

Furthermore, preferably, the process of the present invention further comprises after the molding step and prior to the closing step the step of sterilizing the small-bore female connector, preferably radio-sterilizing the small-bore female connector.

## Claims

1. A small-bore female connector suitable for being inserted into a flexible tube, wherein the small-bore female connector comprises
- a hollow body part **(1),**
- a first removable part **(2),** and
- a second removable part **(3),**
wherein the hollow body part **(1)** comprises
- a device-side part **(4)** comprising a small-bore female connector part **(5)** having a small-bore female opening **(6),** and
- a tube-side part **(7)** comprising a tube engaging part **(8)** having a fluid receiving opening **(9),**
wherein the first removable part **(2)** forms a tube-side connection **(10)** to the tube-side part **(7)** of the hollow body part **(1),** thereby breakably closing the fluid receiving opening **(9),** and
wherein the second removable part **(3)** forms a device-side connection **(11)** to the device-side part **(4)** of the hollow body part **(1).**

2. The small-bore female connector according to claim 1, wherein the second removable part **(3)** forming a device-side connection **(11)** to the device-side part **(4)** of the hollow body part **(1)** results in breakably closing the small-bore female opening **(6).**

3. The small-bore female connector according to any of the preceding claims 1 or 2, wherein the tube engaging part **(8)** comprises an engaging section **(8a)** for engaging with the tube and a tapered, preferably conical, section **(8b)** for centering the tube,
wherein the engaging section **(8a)** connects the tube engaging part **(8)** to the hollow body part **(1),** and
wherein the tapered part **(8b)** connects the engaging section **(8a)** with the first removable part **(2).**

4. The small-bore female connector according to any of the preceding claims 1 to 3, wherein the hollow body part **(1)** further comprises a radially extending flange **(12).**

5. The small-bore female connector according to claim 4, wherein the radially extending flange **(12)** is positioned at a connection between the tube-side part **(7)** and the device-side part **(4).**

6. The small-bore female connector according to any of the preceding claims 1 to 5, wherein the hollow body part **(1)** comprises a hole **(13)** extending through the hollow body part **(1)** connecting the small-bore female opening **(6)** and the fluid receiving opening **(9).**

7. The small-bore female connector according to any of the preceding claims 1 to 6, wherein the first removable part **(2)** comprises at least two lateral extensions **(14).**

8. The small-bore female connector according to claim 7, wherein the breadth of the at least two lateral extensions **(14)** decreases along the lateral dimension of the at least two lateral extensions **(14)** from the tube-side part **(7)** towards an end of the first removable part **(2).**

9. The small-bore female connector according to claims 7 or 8, wherein the second removable part **(3)** comprises at least two lateral extensions **(15)** for gripping said second removable part **(3)** and applying force or torque to twist it off.

10. The small-bore female connector according to any of the preceding claims 1 to 9, wherein the small-bore female connector further comprises a retaining string **(17),** wherein the retaining string **(17)** is connected to the second removable part **(3)** and the hollow body part **(1),** preferably the flange **(12)** of the hollow body part **(1).**

11. The small-bore female connector according to any of the preceding claims 1 to 10, wherein the small-bore female connector further comprises a cap **(18)** suitable for being placed on the small-bore female connector part **(5)** after twisting off the second removable part **(3).**

12. The small-bore female connector according to claim 11, wherein the cap **(18)** is connected to the second removable part **(3).**

13. Use of the small-bore female connector according to any of the preceding claims 1 to 12 in a pharmaceutical product, preferably as a port in a pharmaceutical product, more preferably as a port in a bag comprising an active pharmaceutical ingredient.

14. Process for preparing a small-bore female connector according to any of the preceding claims 1 to 12, wherein the process comprises the steps of:
- injection molding the small-bore female connector thereby shaping the tube-side connection **(10)** and the device-side connection **(11);**
- closing the twist-off opening **(15)** of the second removable part **(3).**

15. The process according to claim 14, wherein the step of closing of the twist-off opening **(16)** comprises the step of inserting a plug into the twist-off opening **(16)** or the step of sealing, preferably thermally sealing, most preferably heating, flattening and welding, the twist-off opening **(16).**
